# EUROPEAN PATENT SPECIFICATION

(11) **EP 2 708 185 B1**
(45) Date of publication and mention of the grant of the patent: **05.04.2017**
(21) Application number: 13382357.5
(22) Date of filing: 17.09.2013
(51) Int. Cl.: A61B 5/11, A61B 5/00, G08B 21/04

(54) **Alarm method and system for detecting incorrect postures**
Alarmverfahren und System zur Detektion falscher Haltungen
Système et procédé d'alarme pour la détection de mauvaises postures

(30) Priority: 17.09.2012 ES 201231433
(43) Date of publication of application: 19.03.2014
(73) Proprietor: Lopez Gonzalez, Mª del Carmen, 28039 Madrid (ES); De Augusto Ramos, Casimiro, 28039 Madrid (ES); Carreno Gil, Cristina, 28039 Madrid (ES); Laosa Alonso, Luis Miguel, 28039 Madrid (ES)
(72) Inventor: De Augusto Ramos, Casimiro, 28039 MADRID (ES); Carreno Gil, Cristina, 28039 MADRID (ES); Laosa Alonso, Luis, Miguel, 28039 MADRID (ES)
(74) Representative: Carpintero Lopez, Francisco

(56) References cited:
- EP-A1- 1 195 139
- WO-A1-2008/059418
- WO-A1-2008/129451
- WO-A1-2010/026513
- US-A1- 2011 025 493

## Description

### Object of the Invention

The present invention is applicable in the heath field, relating to postural education in patients and correcting incorrect postures that they unintentionally adopt. Specifically, it relates to an alarm method which allows notifying the patient when he/she adopts an incorrect posture such that he/she can correct same to prevent subsequent injuries or ailments.

### Background of the Invention

Nowadays, developed societies are experiencing a continuous increase in ailments and injuries caused by unintentionally adopting poor postures. The increasing sedentary lifestyle and the number of hours spent sitting in front of a computer, for example, can have serious repercussions on the health and well-being of affected people.

The most significant consequences suffered as a result of adopting poor body posture for a prolonged time period could lead to a greater risk of injuries; higher possibilities of having a protruding stomach as a result of a curved spine resulting in the weakening of abdominal muscles; increased neck and back pains as a result of pressures exerted on the neck and back; as well as a poor appearance since a straight back transmits a more elegant and slender image than a hunched figure.

Adopting unsuitable postures can also lead to a lack of concentration and therefore reduced productivity. So it is not only an important subject in terms of health but also in terms of productivity and performance.

These reasons make posture health an issue of utmost importance in modern societies. It has conventionally been dealt with by means of recommendations establishing certain guidelines for adopting a correct position, but it is common for one to unintentionally and quickly adopt a posture that is not recommended. In response to this problem, a number of mechanical solutions have been proposed for keeping the back straight or generally for maintaining correct position, from special chairs, stabilizing rods, neck supports...all kinds of devices limiting the movements of the user for keeping him/her in a correct position such as the device proposed in patent CN2877635U, for example. Their general problem is that they are rigid and rather inflexible, besides being quite cumbersome.

Another kind of more modern solutions focus on allowing the user complete freedom in his/her movements, being limited to monitoring the data thereof for establishing, if necessary, an action protocol such as patent TW201108989, for example, which uses the readings from an inertial measuring device to record a possible fall of a user. Another example is patent WO 2009/090509, which records the dynamic parameters of a user while walking. The solution proposed by the document EP1195139 A1 also refers to body moving monitoring. It is able to evaluate the falling risk and walking/running periods focusing on postural transitions. It is targeted to old people or patients already susceptible to suffer a fall, but not working in real time but as a result of a long period observation. All the measurements are stored and sent to a computer for further analysis and if necessary, warning a third person to act.

In the same line of fall detection and/or prevention methods, it can be regarded the solution proposed by document WO2008/0591418 A1, where it is disclosed an invention for fall prevention which it is conceived to attach a number of sensors to a lower body segment, since obtaining some measures of said body segment allows the invention to compare the sequence of postures with a predetermined sequence of postures to determine if there is a risk of a fall. Also the document WO2010/026513 A1 proposes a solution in the same line, which indeed means an improvement over WO2008/0591418 A1. It is referred to the same system for fall prevention but including a more complex computation which estimates the trajectory of the centre of mass of the user relative to a trajectory of a lower portion of the body from the signal or signals.

Another solution of prior art is provided by document WO2008/129451 A1, which refers to a fall detection system. It comprises sensors to be worn by the user and for collecting motion data of the user. The main contribution of this solution lies in the detection of fake alarms, since it makes an evaluation of the sensors to determine if an alarm is a fake alarm by detecting a full rotation of the sensors, a free falling phase or a certain proximity to the body of the user. Therefore, this solution is not actually a prevention method but a way to know if, once a fall has already occurred, the alarm may be a fake alarm or it is a real alarm.

In general, such solutions lack a specific application in the posture health field and the use thereof does not extend beyond collecting subsequent information. Therefore, according to the foregoing the state of the art includes several solutions which attempt to correct the posture of an individual but it lacks a simple, immediate-acting, flexible and effective solution which allows the user to maintain correct posture for his/her health at all times.

### Description of the Invention

The present invention relates to an alarm method for detecting incorrect postures of a user and thus preventing typical muscle ailments or injuries caused by unintentionally adopting poor posture for a prolonged time period. The proposed invention comprises the steps of:
- Recording a static initial position of a user in a processing module. This provides the invention with the flexibility necessary to enable adapting the invention to anyone.
- Obtaining inertial measurements with respect to the static initial position through inertial sensors, recording said measurements as soon as the user performs a movement.
- Obtaining from the signals coming from the inertial sensors the deviation of the position of the user with respect to the static initial position in the processing module, being said deviation the angle of inclination of the user with respect to the static initial position.
- Verifying if the deviation is greater than a pre-established limit to allow a certain range of movements in which the posture of the user is not considered deviated enough so as to necessitate correction.
- Activating an alarm if the verification of the preceding step shows that the deviation is greater than the pre-established limit for a certain time period. In response to this alarm, the reaction of the user will be to correct his/her posture until returning to his/her initial posture.

It is contemplated that the inertial measurements recorded by the system of the invention comprise the acceleration and angular velocity of the user. Furthermore, the deviation of the user with respect to his/her initial position can be measured as the angle of inclination with respect to the initial position.

An additional step consisting of sending a wireless signal to a wireless alarm device can optionally be included.

Another aspect of the invention comprises an alarm system for detecting incorrect postures of a user according to claim 5.

In one embodiment of the invention, it is contemplated that the deviation module comprises an accelerometer measuring the acceleration of the user and a gyroscope measuring the angular velocity.

The alarm module can comprise a vibrator and a bell.

The invention can comprise in one of its embodiments a wireless communication module transmitting a wireless alarm signal to a wireless device linked to the system.

The configuration module can comprise a plurality of buttons for configuring the limit beyond which the posture of the user is considered incorrect, the time period after which the alarm is triggered, configuring the type of alarm and configuring the communication with other devices. It can also comprise a display where the chosen options are shown.

The system can comprise in one of its embodiments one or several LED diodes indicating the chosen configuration.

The proposed invention can be placed in various supports to be transported by the user. One of the possible embodiments of the system comprises a belt where the proposed system is housed by way of a buckle.

Therefore, according to the described invention the alarm method proposed by the invention is an improvement in the methods for preventing neck and back injuries due to poor posture that have been used up until now, and it solves the aforementioned problem in a fully satisfactory manner on the basis of replacing rigid, rather inadaptable and much more complex systems, with the subsequent cost reduction due to process optimization and efficiency, all this through a simple design which does not require any special skill or training for use.

### Description of the Drawings

To complement the description being made and for the purpose of aiding to better understand the features of the invention according to a preferred practical embodiment thereof, a set of drawings is attached as an integral part of said description in which the following has been depicted with an illustrative and non-limiting character:
Figure 1 shows the preferred embodiment of the system inserted in a belt by way of a buckle.
Figure 2 shows the system of the invention in detail.
Figure 3 shows the different modules comprised in the invention.
Figure 4 shows a flow chart of the processing module of one of the embodiments.

### Preferred Embodiment of the Invention

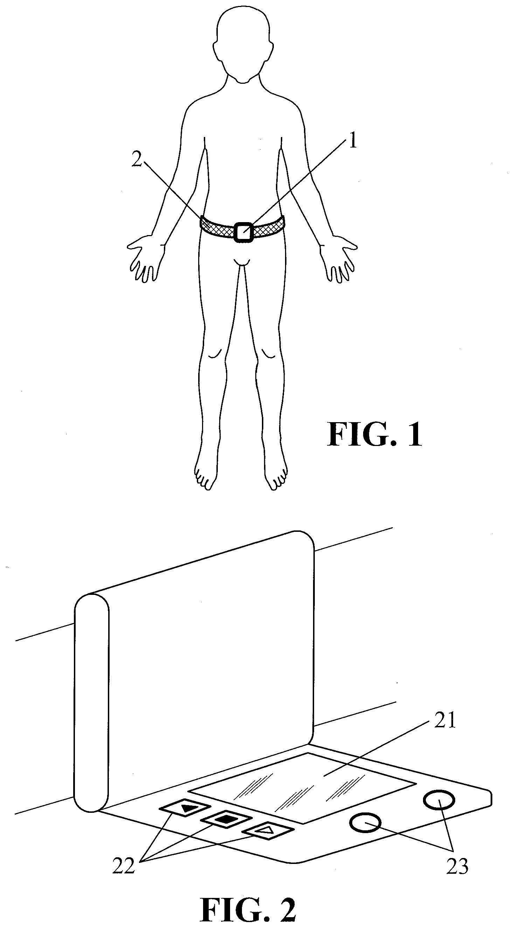

In view of the described drawings, it can be seen how in one of the possible embodiments of the invention, which can be named a "digital posture re-educator", it consists of an electronic device which allows the placement thereof in different supports, for example, in a belt (2) by way of a buckle (1) as shown in Figure 1, by means of an adaptor.

Figure 2 show an embodiment of the device used. The device shown is open and the keypad (22) used for introducing the configurations and establishing the deviation limit, the time period after which the alarm is triggered and the type of alarm, can be seen. Figure 2 also shows the display (21) and the LED diodes (23) where the chosen configuration options are depicted.

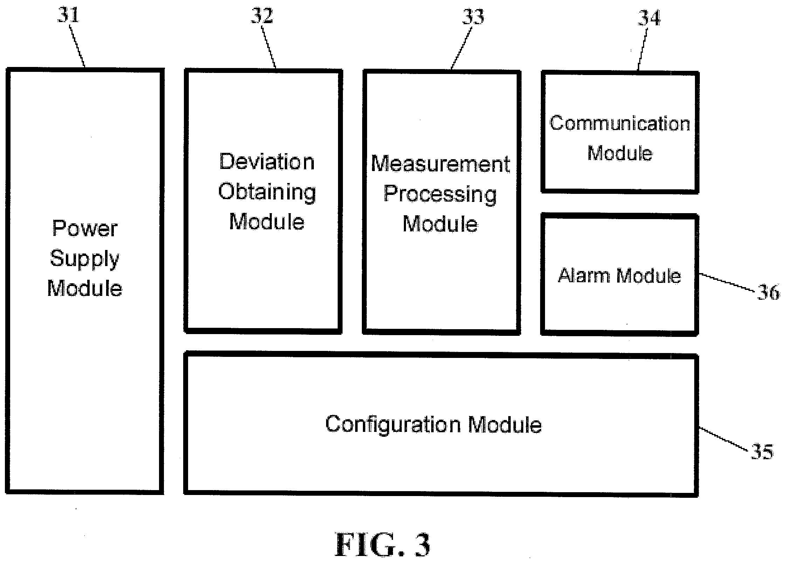

The preferred embodiment of the system of the invention comprises the following modules depicted in Figure 3:
a) Power supply module (31): it comprises a battery providing direct current and voltage to all the electronic components forming the system. For example, a lithium polymer battery providing the system, in one of its embodiments, a constant voltage of 3.7 V. The battery is ideally rechargeable, for which purpose the module comprises a circuit which allows charging the battery through a mini USB port (as is done today with mobile telephones or MP3 devices). The battery charge level is shown by means of a multicolor LED diode allowing the user to know the state of the battery.
b) Deviation obtaining module (32): this module provides information about the deviation of the user, a measurement such as the angle of inclination, with respect to the initial position, which is considered as correct. Said information is collected as a result of an inertial measuring unit (IMU) which is the main component of the module. The IMU comprises an accelerometer and a gyroscope, which allows obtaining measurements on 5 axes (Roll, Pitch, X, Y, Z).

The signal given by the gyroscope is the angular velocity with which the device is rotating with respect to a fixed reference system.

The signal given by the accelerometer is the acceleration produced when a movement occurs in each of its axes. The accelerometer can also be used as an inclination sensor if gravity acceleration is used as a vector for determining the orientation of the device.

One of the most important aspects is that the calculation of the deviation of the user with respect to the initial position must be as true as possible since the measurements taken by the inertial sensors are not very accurate over long time intervals due to the gradual accumulation of minor errors. Said errors are corrected by the combined use of the accelerometer and gyroscope.
c) Measurement processing module (33): it processes the measurements obtained by the deviation obtaining module. It comprises a microprocessor which analyzes the input signals coming from the deviation obtaining module that reach it in the form of voltage variations, and it converts them into degrees for obtaining the angle of inclination of the system therefrom.
   This module can include a Kalman filter to compensate for the vibration of the entire system. The outputs of the accelerometer and gyroscope are filtered such that the measurement is more reliable.
d) Alarm module (34): it notifies the user by means of a light, sound and/or vibrating system depending on the configuration selected by the user since it has a LED diode, a vibrator and a bell-loudspeaker.
e) Communication module (36): it sends an alarm signal to a device linked to the system using wireless technology such as Bluetooth, for example, depending on the configuration selected by the user.
f) Configuration module (35): it comprises a display, a keypad and LED diodes. This module allows the user to configure the following system settings:
   - Sensitivity: it allows configuring the deviation limit of the user with respect to the initial position. Several levels of sensitivity can be established: high, medium or low. Depending on the selected sensitivity, the device will allow the user more or less deviation from his/her posture.
   - Zero-position light setting: it emits a light signal by means of a LED diode to guide the user to adopt a correct position, bringing the user to the initial position.
   - Incorrect posture notification mode: it allows selecting from the following types of notification: vibration, sound or both.
   - Time: it allows setting the maximum time allowed in an incorrect position before triggering an alarm.
   - Transmission to another device: it allows selecting the activation/deactivation of the wireless signal and linking with the device to which the alarm signal is transmitted.

The step-by-step operation of the invention consists of first recording the initial position of the user. Once that initial position, which is considered as correct, is known, the objective is for the user to not unintentionally lose said posture such that future discomforts or injuries can be prevented. The next step consists of obtaining inertial measurements by means of an inertial measuring unit comprising an accelerometer and a gyroscope. Therefore, if the user performs any movement with respect to the initial position, the invention obtains the deviation with respect to said initial position, measured as the angle of inclination.

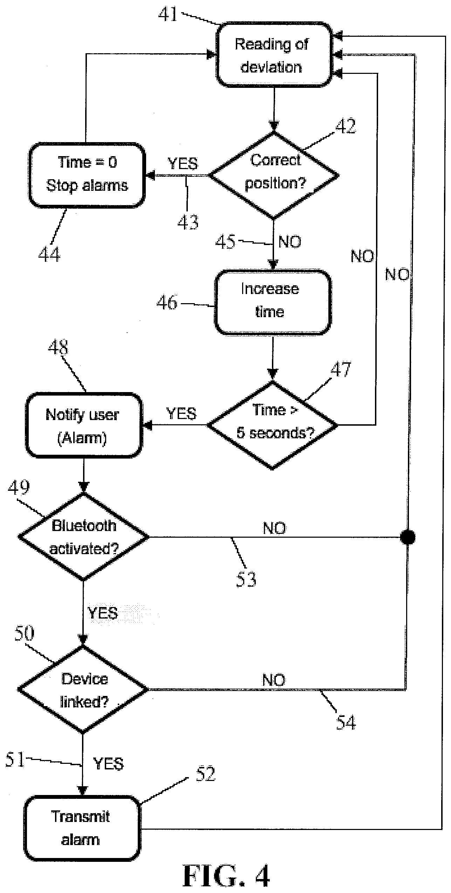

Then, the method followed in the processing module can be seen in Figure 4. It consists of a loop which, in permanent contact with the deviation obtaining module (32), takes constant readings (41) of the deviation provided by the deviation obtaining module. These deviations are compared (42) with a pre-established limit to evaluate whether they are correct (43), i.e., they do not exceed said limit, or incorrect (45), i.e., they exceed said limit. As long as the position is correct no action is taken except resetting (44) the wait time to zero and waiting for the next reading of the deviation. In contrast, however, if the position is not considered correct, then the wait time starts to increase (36). The wait time continues to increase with each consecutive reading (41) of the deviation which is considered as an incorrect position until reaching the pre-established limit (47), at which time the processing module sends a communication (48) to the alarm module so that it triggers the alarm that is configured, be it a light, sound or vibrating alarm. If the Bluetooth signal 49 is active 50 and there is a device linked 51 to the system, the alarm 52 is transmitted to said device. If Bluetooth is not activated 53 or there is no linked device 54, the system returns to step 41 and continues with the readings.

## Claims

1. Alarm method for detecting incorrect postures of a user, comprising the following steps:
- recording a static initial position of a user in a processing module;
- obtaining inertial measurements with respect to the static initial position through inertial sensors;
- obtaining from the signals coming from the inertial sensors the deviation of the position of the user with respect to the static initial position in the processing module, being said deviation the angle of inclination of the user with respect to the static initial position;
- verifying if the deviation is greater than a pre-established limit;
- activating an alarm if the verification of the previous step shows that the deviation is greater than the pre-established limit for a certain time period.

2. Method according to the previous claim, wherein the inertial measurements comprise the acceleration and angular velocity of the user.

3. Method according to any of the previous claims, further comprising sending a wireless signal to an alarm device.

4. Method according to claim 3, wherein the wireless signal is transmitted by means of Bluetooth technology.

5. Alarm system for detecting incorrect postures of a user, comprising
- a power supply module providing constant voltage to the system;
- a deviation module comprising inertial sensors for obtaining inertial measurements;
- a processing module recording a static initial position of the user and calculating the deviation from said static initial position from the data offered by the deviation module, being said deviation the angle of inclination of the user with respect to the static initial position, verifying if the deviation is greater than a pre-established limit once calculated and communicating it to an alarm module;
- an alarm module notifying the user if it receives communication from the processing module;
- a configuration module where the user establishes the type of alarm and the limit for the deviation.

6. System according to the previous claim, wherein the deviation module comprises an accelerometer measuring the acceleration of the user and a gyroscope measuring the angular velocity.

7. System according to the previous claim, wherein the alarm module comprises a vibrator and a bell.

8. System according to any of claims 5-7, wherein the configuration module further comprises a display and a plurality of buttons for configuring the deviation limit, the time period after which the alarm is triggered and the type of alarm.

9. System according to any of the previous claims, further comprising a wireless communication module transmitting a wireless alarm signal to a wireless device linked to the system.

10. System according to the previous claim, wherein the configuration module further comprises at least one LED diode indicating the chosen configuration.

11. System according to any of claims 5-10, wherein the entire system is housed in a belt by way of a buckle.

## Patentansprüche

1. Ein Alarmverfahren zur Detektion einer fehlerhaften Körperhaltung eines Anwenders umfassend die folgenden Schritte:
- Aufnahme einer statischen Anfangsposition eines Anwenders im Verarbeitungsmodul;
- Erhalten von Trägheitsmessungen in Bezug auf die statische Anfangsposition mittels Trägheitssensoren;
- Erhalten der Abweichung der Position des Anwenders in Bezug auf die statische Anfangsposition in dem Verarbeitungsmodul von von den Trägheitssensoren kommenden Signalen, wobei die Abweichung der Neigungswinkel des Anwenders in Bezug auf die statische Anfangsposition ist;
- Bestätigen, ob die Abweichung größer ist als eine vorab festgesetzte Grenze;
- Aktivieren eines Alarms, wenn die Bestätigung des vorhergehenden Schritts zeigt, dass die Abweichung größer ist als die vorab festgesetzte Grenze für einen bestimmten Zeitraum.

2. Verfahren gemäß dem vorhergehenden Anspruch, wobei die Trägheitsmessungen die Beschleunigung und Winkelgeschwindigkeit des Anwenders umfasst.

3. Verfahren gemäß einem der vorhergehenden Ansprüche, des Weiteren das Aussenden eines drahtlosen Signales zu einer Alarmvorrichtung umfassend.

4. Verfahren gemäß Anspruch 1, wobei das drahtlose Signal mittels Bluetooth-Technologie übertragen wird.

5. Alarmsystem zum Detektieren von fehlerhaften Körperhaltungen eines Anwenders, umfassend
- ein Stromversorgungsmodul zur Bereitstellung einer konstanten Spannung für das System;
- ein Abweichungsmodul umfassend Trägheitssensoren zum Erhalten von Trägheitsmessungen;
- ein Verarbeitungsmodul, das eine statische Anfangsposition des Anwenders aufnimmt und die Abweichung von der statischen Anfangsposition von den Daten, angezeigt von dem Abweichungsmodul, berechnet, wobei die Abweichung der Neigungswinkel des Anwenders in Bezug auf die statische Anfangsposition ist; Bestätigen, dass die Abweichung größer ist als eine vorab festgesetzte Grenze, wenn einmal berechnet, und deren Kommunikation an ein Alarmmodul;
- ein Alarmmodul, welches den Anwender benachrichtigt, wenn es die Kommunikation von dem Verarbeitungsmodul empfängt;
- ein Konfigurationsmodul, wobei der Anwender den Alarmtyp und die Grenze der Abweichung bestimmt.

6. System gemäß dem vorhergehenden Anspruch, wobei das Abweichungsmodul einen Beschleuniger umfasst, der die Beschleunigung des Anwenders misst, und ein Gyroskop umfasst, das die Winkelgeschwindigkeit misst.

7. System gemäß dem vorhergehenden Anspruch, wobei das Alarmmodul einen Vibrator und eine Klingel umfasst.

8. System gemäß einem der Ansprüche 5 bis 7, wobei das Konfigurationsmodul des Weiteren eine Anzeige und eine Vielzahl von Tasten zur Konfiguration der Abweichungsgrenze, des Zeitraums, nach welchem Alarm ausgelöst wird und der Art des Alarms umfasst.

9. System gemäß einem der vorhergehenden Ansprüche, des Weiteren ein drahtloses Kommunikationsmodul umfassend, das ein drahtloses Alarmsignal zu einer drahtlosen Vorrichtung, die mit dem System verbunden ist, überträgt.

10. System gemäß dem vorhergehenden Anspruch, wobei das Konfigurationsmodul des Weiteren mindestens eine LED-Diode umfasst, die die gewählte Konfiguration anzeigt.

11. System gemäß einem der Ansprüche 5 bis 10, wobei das gesamte System in einem Gürtel in einer Schnalle untergebracht ist.

## Revendications

1. Procédé d'alarme pour la détection de mauvaises postures d'un utilisateur, comprenant les étapes suivantes :
- enregistrer une position initiale statique d'un utilisateur dans un module de traitement ;
- obtenir des mesures inertielles en ce qui concerne la position initiale statique par l'intermédiaire de capteurs inertiels ;
- obtenir à partir des signaux provenant des capteurs inertiels l'écart de la position de l'utilisateur par rapport à la position initiale statique dans le module de traitement, l'écart étant l'angle d'inclinaison de l'utilisateur par rapport à la position initiale statique ;
- vérifier si l'écart est supérieur à une limite préétablie ;
- activer une alarme si la vérification de l'étape précédente montre que l'écart est supérieur à la limite préétablie pendant une certaine période de temps.

2. Procédé selon la revendication précédente, dans lequel les mesures inertielles comprennent l'accélération et la vitesse angulaire de l'utilisateur.

3. Procédé selon l'une quelconque des revendications précédentes, comprenant en outre l'envoi d'un signal sans fil à un dispositif d'alarme.

4. Procédé selon la revendication 3, dans lequel le signal sans fil est transmis au moyen de la technologie Bluetooth.

5. Système d'alarme pour la détection de mauvaises postures d'un utilisateur, comprenant :
- un module d'alimentation fournissant une tension constante au système ;
- un module d'écart comprenant des capteurs inertiels pour obtenir des mesures inertielles ;
- un module de traitement enregistrant une position initiale statique de l'utilisateur et calculant l'écart par rapport à la position initiale statique à partir des données fournies par le module d'écart, l'écart étant l'angle d'inclinaison de l'utilisateur par rapport à la position initiale statique, vérifiant si l'écart est supérieur à une limite préétablie une fois calculé et communiquant celui-ci à un module d'alarme ;
- un module d'alarme informant l'utilisateur s'il reçoit une communication provenant du module de traitement ;
- un module de configuration dans lequel l'utilisateur établit le type d'alarme et la limite pour l'écart.

6. Système selon la revendication précédente, dans lequel le module d'écart comprend un accéléromètre mesurant l'accélération de l'utilisateur et un gyroscope mesurant la vitesse angulaire.

7. Système selon la revendication précédente, dans lequel le module d'alarme comprend un vibreur et une sonnerie.

8. Système selon l'une quelconque des revendications 5 à 7, dans lequel le module de configuration comprend un outre un afficheur et une pluralité de boutons pour configurer la limite d'écart, la période de temps après laquelle l'alarme est déclenchée et le type d'alarme.

9. Système selon l'une quelconque des revendications précédentes, comprenant en outre un module de communication sans fil transmettant un signal d'alarme sans fil à un dispositif sans fil relié au système.

10. Système selon la revendication précédente, dans lequel le module de configuration comprend en outre au moins une diode LED indiquant la configuration choisie.

11. Système selon l'une quelconque des revendications 5 à 10, dans lequel le système entier est logé dans une ceinture au moyen d'une boucle de ceinture.
